(19) Europäisches Patentamt / European Patent Office / Office européen des brevets

(11) **EP 4 737 507 A1**

(12) **EUROPEAN PATENT APPLICATION**
published in accordance with Art. 153(4) EPC

(43) Date of publication:
06.05.2026 Bulletin 2026/19

(21) Application number: 24831832.1

(22) Date of filing: 20.06.2024

(51) International Patent Classification (IPC):
*C08J 3/12* (2006.01)     *A61F 13/15* (2006.01)
*A61F 13/53* (2006.01)

(52) Cooperative Patent Classification (CPC):
A61F 13/15; A61F 13/53; C08J 3/12

(86) International application number:
PCT/JP2024/022475

(87) International publication number:
WO 2025/004971 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.06.2023 JP 2023107786

(71) Applicant: Sumitomo Seika Chemicals Co., Ltd.
Kako-gun, Hyogo 675-0145 (JP)

(72) Inventors:
• AWAJI, Yuki
Himeji-shi, Hyogo 672-8076 (JP)
• HIRANO, Saho
Himeji-shi, Hyogo 672-8076 (JP)

(74) Representative: Mewburn Ellis LLP
Aurora Building
Counterslip
Bristol BS1 6BX (GB)

(54) **METHOD FOR PRODUCING WATER-ABSORBING RESIN PARTICLES, WATER-ABSORBING RESIN PARTICLES, ABSORBER, AND ABSORBENT ARTICLE**

(57) There is provided a novel method for producing water-absorbent resin particles having gel stability against artificial urine containing iron, long-term gel stability against artificial urine, and yellowing resistance. A method for producing water-absorbent resin particles, comprising the steps of polymerizing a water-soluble ethylenically unsaturated monomer to obtain polymer particles; and subjecting the polymer particles to surface-crosslinking, wherein before and/or after the step of surface-crosslinking, a phosphonic acid-based chelating agent is added to the polymer particles having a water content of 5% by mass or more and 100% by mass or less, and wherein an amount of the phosphonic acid-based chelating agent added is 0.055 parts by mass or more and 0.6 parts by mass or less per 100 parts by mass of the water-soluble ethylenically unsaturated monomer.

**EP 4 737 507 A1**

**Description**

Technical Field

The present invention relates to a method for producing water-absorbent resin particles, water-absorbent resin particles, an absorbent material, and an absorbent article; and more particularly relates to a method for producing water-absorbent resin particles that constitute an absorbent material suitably used for hygienic materials, such as disposable diapers, sanitary napkins, and incontinence pads, water-absorbent resin particles, an absorbent material including the water-absorbent resin particles, and an absorbent article.

Background Art

In recent years, water-absorbent resin particles have been widely used in the field of hygienic materials, such as disposable diapers, sanitary napkins, and incontinence pads.

As such water-absorbent resin particles, crosslinked products of polymers of a water-soluble ethylenically unsaturated monomer, more specifically, crosslinked products of polymers of partially neutralized polyacrylic acid, have been proposed as preferable water-absorbent resin particles, because of many advantages: for example, they have good water absorption capacity; acrylic acid used as a raw material thereof is readily industrially available, and thus, they can be produced at low cost with uniform quality; additionally, they are resistant to decomposition or degradation (see, for example, Patent Literature 1).

An absorbent article, such as a disposable diaper, a sanitary napkin, or an incontinence pad, is mainly composed of an absorbent material that is positioned in a central portion, and absorbs and retains a body liquid, such as urine or menses excreted from the body; a liquid-permeable front sheet (top sheet) positioned on the side of the absorbent article that is brought into contact with the body; and a liquid-impermeable rear sheet (back sheet) positioned opposite to the side that is brought into contact with the body. The absorbent material is typically composed of hydrophilic fibers, such as pulp, and water-absorbent resin particles.

Citation List

Patent Literature

Patent Literature 1: JP-A-H3-227301

Summary of Invention

Technical Problem

In such an absorbent article, the water-absorbent resin particles contained in the absorbent material may be affected by vitamin C in urine and iron contained in urine or in the water-absorbent resin particles, which may cause a phenomenon in which the gel deteriorates.

A method for inhibiting the gel deterioration of the water-absorbent resin particles as described above is known, in which a metal chelating agent is added in the production process of the water-absorbent resin particles. For example, an aminocarboxylic acid-based chelating agent coordinates to iron or the like, and exhibits the effect of inhibiting the deterioration of the water-absorbent resin particles due to vitamin C and iron.

Furthermore, the aminocarboxylic acid-based chelating agent not only inhibits the gel deterioration of the water-absorbent resin particles, but also exhibits the effect of inhibiting yellowing of the water-absorbent resin particles.

However, the inventors of the present invention have found that when urine has a high iron concentration, the effect of inhibiting the gel deterioration by the aminocarboxylic acid-based chelating agent is not sufficient.

Under such circumstances, it is a main object of the present invention to provide a novel method for producing water-absorbent resin particles having gel stability against artificial urine containing iron, long-term gel stability against artificial urine, and yellowing resistance. It is also an object of the present invention to provide water-absorbent resin particles having high absorption performance (particularly in terms of physiological saline retention capacity and physiological saline absorption capacity under a load of 4.14 kPa) and also having gel stability against artificial urine containing iron, long-term gel stability against artificial urine, and yellowing resistance, an absorbent material including the water-absorbent resin particles, and an absorbent article.

Solution to Problem

The present inventors have conducted extensive research to solve the aforementioned problem. As a result, the inventors have found that water-absorbent resin particles having gel stability against artificial urine containing iron, long-term gel stability against artificial urine, and yellowing resistance can be obtained using a method for producing water-absorbent resin particles, comprising the steps of polymerizing a water-soluble ethylenically unsaturated monomer to obtain polymer particles; and subjecting the polymer particles to surface-crosslinking, wherein at at least one timing from before and after the step of surface-crosslinking, a predetermined amount of a phosphonic acid-based chelating agent is added to the polymer particles adjusted to a predetermined water content. The present invention has been accomplished as a result of further research based on this finding.

In summary, the present invention provides aspects of the invention comprising the following features:

Item 1. A method for producing water-absorbent resin particles, comprising the steps of:

polymerizing a water-soluble ethylenically unsaturated monomer to obtain polymer particles; and
subjecting the polymer particles to surface-crosslinking,
wherein before and/or after the step of surface-crosslinking, a phosphonic acid-based chelating agent is added to the polymer particles having a water content of 5% by mass or more and 100% by mass or less, and
wherein an amount of the phosphonic acid-based chelating agent added is 0.055 parts by mass or more and 0.6 parts by mass or less per 100 parts by mass of the water-soluble ethylenically unsaturated monomer.

Item 2. The method according to item 1, wherein the water content in the polymer particles is 20% by mass or more and 80% by mass or less.

Item 3. The method according to item 1 or 2, wherein the water-absorbent resin particles have the following properties (A) to (E):

(A) a physiological saline retention capacity of 20 g/g or more and 70 g/g or less;
(B) a physiological saline absorption capacity under a load of 4.14 kPa of 5 mL/g or more and 40 mL/g or less;
(C) a yellowness index of less than 25 after allowing the water-absorbent resin particles to stand for 7 days in an environment of 70°C and 90% relative humidity;
(D) a gel strength of 6000 $N/m^2$ or more after allowing a gel swollen 40-fold with artificial urine to stand for 14 hours in an environment of 37°C and 60% relative humidity; and
(E) a gel strength of 4000 $N/m^2$ or more after allowing a gel swollen 40-fold with artificial urine containing iron at an iron concentration of 10 ppm to stand for 2.5 hours in an environment of 25°C and 50% relative humidity.

Item 4. Water-absorbent resin particles, the water-absorbent resin particles being a crosslinked product of a polymer of a water-soluble ethylenically unsaturated monomer,
wherein the water-absorbent resin particles have the following properties (A) to (E):

(A) a physiological saline retention capacity of 20 g/g or more and 70 g/g or less;
(B) a physiological saline absorption capacity under a load of 4.14 kPa of 5 mL/g or more and 40 mL/g or less;
(C) a yellowness index of less than 25 after allowing the water-absorbent resin particles to stand for 7 days in an environment of 70°C and 90% relative humidity;
(D) a gel strength of 6000 $N/m^2$ or more after allowing a gel swollen 40-fold with artificial urine to stand for 14 hours in an environment of 37°C and 60% relative humidity; and
(E) a gel strength of 4000 $N/m^2$ or more after allowing a gel swollen 40-fold with artificial urine containing iron at an iron concentration of 10 ppm to stand for 2.5 hours in an environment of 25°C and 50% relative humidity.

Item 5. An absorbent material comprising the water-absorbent resin particles according to item 4.

Item 6. An absorbent article comprising the absorbent material according to item 5.

Advantageous Effects of Invention

According to the present invention, it is possible to provide a novel method for producing water-absorbent resin particles having gel stability against artificial urine containing iron, long-term gel stability against artificial urine, and yellowing resistance. Furthermore, according to the present invention, it is also possible to provide water-absorbent resin particles having high absorption performance (particularly in terms of physiological saline retention capacity and physiological saline absorption capacity under a load of 4.14 kPa) and also having gel stability against artificial urine containing iron, long-term gel stability against artificial urine, and yellowing resistance, an absorbent material including the water-absorbent resin particles, and an absorbent article.

Brief Description of Drawings

Fig. 1 is a schematic diagram of a measurement apparatus for physiological saline absorption capacity under a load of 4.14 kPa.
Fig. 2 is a schematic diagram of a measurement apparatus used for measurement of gel strength.

Description of Embodiments

As used herein, the term "comprising" includes "consisting essentially of" and "consisting of". As used herein, the term "(meth)acrylic" refers to "acrylic or methacrylic", the term "(meth)acrylate" refers to "acrylate or methacrylate", and the term "(poly)" means both cases with and without the prefix "poly". As used herein, the term "water-soluble" refers to having a water solubility of 5% by mass or more at 25°C.
As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

1. Method for Producing Water-Absorbent Resin Particles

A method for producing water-absorbent resin particles according to the present invention comprises the steps of polymerizing a water-soluble ethylenically unsaturated monomer to obtain polymer particles; and subjecting the polymer particles to surface-crosslinking, in the mentioned order.
In the method for producing water-absorbent resin particles of the present invention, at least one of before and after the step of surface-crosslinking, a phosphonic acid-based chelating agent is added to the polymer particles having a water content of 5% by mass or more and 100% by mass or less. Furthermore, an amount of the phosphonic acid-based chelating agent then added is in a range of 0.055 parts by mass or more and 0.6 parts by mass or less per 100 parts by mass of the water-soluble ethylenically unsaturated monomer.
Because of these features, the method for producing water-absorbent resin particles of the present invention can suitably produce water-absorbent resin particles having gel stability against artificial urine containing iron, long-term gel stability against artificial urine, and yellowing resistance. The method for producing water-absorbent resin particles of the present invention will be hereinafter described in detail.

<Polymerization Step>

The polymerization step is the step of polymerizing a water-soluble ethylenically unsaturated monomer to obtain polymer particles. Examples of methods of polymerizing a water-soluble ethylenically unsaturated monomer include representative polymerization methods such as aqueous polymerization, spray droplet polymerization, emulsion polymerization, and reversed phase suspension polymerization. In aqueous polymerization, polymerization is performed by heating a water-soluble ethylenically unsaturated monomer solution, optionally with stirring. In reversed phase suspension polymerization, polymerization is performed by heating the water-soluble ethylenically unsaturated monomer with stirring in a hydrocarbon dispersion medium. Preferred among the above-mentioned methods is, for example, reversed phase suspension polymerization, in view of improving general water absorption performance (in terms of physiological saline retention capacity and physiological saline absorption capacity under a load of 4.14 kPa) of the water-absorbent resin particles. In the polymerization step, an internal-crosslinking agent may be optionally added to the water-soluble ethylenically unsaturated monomer to obtain crosslinked polymer particles (hydrous gel) having an internal-crosslinked structure. An example of the polymerization step will be hereinafter described.

[Water-Soluble Ethylenically Unsaturated Monomer]

Examples of the water-soluble ethylenically unsaturated monomer include (meth)acrylic acid ("acrylic" and "methacrylic" are herein collectively referred to as "(meth)acrylic"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethylacrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resin particles. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer as an aqueous solution may be dispersed in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturated concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization process. The above-mentioned alkaline neutralizing agents may be used alone or in combinations of two or more.

The neutralization degree of the water-soluble ethylenically unsaturated monomer with an alkaline neutralizing agent, calculated as the neutralization degree of all acid groups in the water-soluble ethylenically unsaturated monomer, is preferably 40 to 100 mol%, more preferably 50 to 90 mol%, still more preferably 60 to 85 mol%, and even more preferably 70 to 80 mol%.

[Radical Polymerization Initiator]

Examples of the radical polymerization initiator added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

[Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N''-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and $\alpha$-methyl epichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.

The amount of the hydrocarbon dispersion medium used is preferably 100 to 1500 parts by mass, and more preferably 200 to 1400 parts by mass, per 100 parts by mass of a first-stage water-soluble ethylenically unsaturated monomer, in view of uniformly dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant used is preferably 0.1 to 30 parts by mass, and more preferably 0.3 to 20 parts by mass, per 100 parts by mass of the first-stage water-soluble ethylenically unsaturated monomer.

(Polymeric Dispersion Agent)

As the dispersion stabilizer to be used in reversed phase suspension polymerization, a polymeric dispersion agent may be used in combination with the above-described surfactant.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent used is preferably 0.1 to 30 parts by mass, and more preferably 0.3 to 20 parts by mass, per 100 parts by mass of the first-stage water-soluble ethylenically unsaturated monomer.

[Other Components]

In the method for producing water-absorbent resin particles, other components may be optionally added to the aqueous solution containing the water-soluble ethylenically unsaturated monomer, which is then subjected to reversed phase suspension polymerization. Various additives such as thickeners and chain transfer agents can be added as the other components.

By way of example, a thickener may be added to the aqueous solution containing the water-soluble ethylenically unsaturated monomer, which is then subjected to reversed phase suspension polymerization. By thus adding a thickener to adjust the viscosity of the aqueous solution, the median particle size obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the water-soluble ethylenically unsaturated monomer solution, the larger the primary particles and/or secondary particles of the resulting particles tend to be.

[Reversed Phase Suspension Polymerization]

Reversed phase suspension polymerization involves dispersing an aqueous monomer solution containing the water-soluble ethylenically unsaturated monomer in a hydrocarbon dispersion medium, in the presence of a dispersion stabilizer, for example. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed and then disperse a surfactant therein, followed by polymerization.

Reversed phase suspension polymerization as described above can be performed in one stage or two or more multiple stages. In view of improving productivity, reversed phase suspension polymerization is preferably performed in two or three stages.

Reversed phase suspension polymerization in two or more multiple stages may be performed as follows: the first stage of reversed phase suspension polymerization is performed; subsequently, the water-soluble ethylenically unsaturated monomer is added to and mixed with the reaction mixture obtained by the first-stage polymerization reaction, and the second and subsequent stages of reversed phase suspension polymerization are performed in the same manner as in the first stage. In each of the second and subsequent stages of reversed phase suspension polymerization, it is preferred to add, in addition to the water-soluble ethylenically unsaturated monomer, a radical polymerization initiator within the above-described range of molar ratios of each component relative to the water-soluble ethylenically unsaturated monomer, based on the amount of the water-soluble ethylenically unsaturated monomer added in each of the second and subsequent stages of reversed phase suspension polymerization, and then perform reversed phase suspension polymerization. In the second and subsequent stages of polymerization, an internal-crosslinking agent may also be optionally added to the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

<Dehydration Step>

After reversed phase suspension polymerization is performed as described above, the method may include a dehydration step of removing the water, the hydrocarbon dispersion medium, and the like by distillation, by adding external energy, such as heat. To remove the water in the hydrous gel after reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. By controlling the treatment conditions for the dehydration step after the polymerization to adjust the amount of water to be removed (i.e., adjust the water content in the polymer particles), various kinds of performance of the resulting water-absorbent resin particles can be controlled. As described above, in the present invention, it is required to control the water content in the polymer particles when the phosphonic acid-based chelating agent is added thereto. In view of more suitably achieving the effects of the present invention, it is also preferred to adjust the water content in the polymer particles to be subjected to the step of surface-crosslinking.

In the dehydration step, the dehydration treatment by distillation may be performed under atmospheric pressure. When the dehydration treatment is performed under atmospheric pressure, the dehydration temperature is preferably 70 to 250°C, more preferably 80 to 180°C, still more preferably 80 to 140°C, and even more preferably 90 to 130°C.

<Step of Surface-Crosslinking>

The step of surface-crosslinking is the step of subjecting the polymer particles obtained in the polymerization step to surface-crosslinking. When the polymer particles are crosslinked polymer particles (a hydrous gel), the step of surface-crosslinking is the step of crosslinking the hydrous gel having an internal-crosslinked structure obtained by polymerization of the water-soluble ethylenically unsaturated monomer, by adding a surface-crosslinking agent thereto (surface-crosslinking reaction). The surface-crosslinking reaction is preferably performed in the presence of a surface-crosslinking agent, after the polymerization of the water-soluble ethylenically unsaturated monomer. By thus subjecting the hydrous gel having an internal-crosslinked structure to the surface-crosslinking reaction after the polymerization, it is possible to obtain water-absorbent resin particles having an increased crosslinking density near the surfaces of the water-absorbent resin particles to exhibit improvement in various kinds of performance, such as water absorption capacity under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxolan-2-one; and hydroxyalkylamide compounds, such as bis[N,N-di($\beta$-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

The amount of the surface-crosslinking agent used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 200 parts by mass of water, still more preferably 10 to 100 parts by mass of water, even more preferably 20 to 60 parts by mass of water, per 100 parts by mass of the water-soluble ethylenically unsaturated monomer. The amount of water herein refers to the total amount of the water contained in the reaction system and the water optionally used during the addition of the surface-crosslinking agent.

In view of more suitably achieving the effects of the present invention, the water content in the polymer particles when the surface-crosslinking agent is added thereto is preferably 1% by mass or more, more preferably 10% by mass or more, and still more preferably 20% by mass or more, while the water content is preferably 60% by mass or less, more preferably 40% by mass or less, and still more preferably 35% by mass or less. Preferred ranges include from 1 to 60% by mass, from 1 to 40% by mass, from 1 to 35% by mass, from 10 to 60% by mass, from 10 to 40% by mass, from 10 to 35% by mass, from 20 to 60% by mass, from 20 to 40% by mass, and from 20 to 35% by mass.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

<Addition of Phosphonic Acid-Based Chelating Agent>

In the method for producing water-absorbent resin particles of the present invention, at least one of before and after the step of surface-crosslinking, a phosphonic acid-based chelating agent is added to the polymer particles. The polymer particles during the addition has a water content of 5% by mass or more and 100% by mass or less. Furthermore, the amount of the phosphonic acid-based chelating agent then added is 0.055 parts by mass or more and 0.6 parts by mass or less per 100 parts by mass of the water-soluble ethylenically unsaturated monomer.

The phosphonic acid-based chelating agent is not specifically limited as long as the effects of the present invention are achieved, and preferred specific examples include ethylenediamine tetramethylene phosphonic acid (EDTMP), diethylenetriamine pentamethylene phosphonic acid (DTPMP), and salts thereof. In view of more suitably achieving the effects of the present invention, among these, the phosphonic acid-based chelating agent preferably includes ethylenediamine tetramethylene phosphonic acid•pentasodium salt (EDTMP•5Na) and diethylenetriamine pentamethylene phosphonic acid·heptasodium salt (DTPMP•7Na), and more preferably includes ethylenediamine tetramethylene phosphonic acid•pentasodium salt (EDTMP•5Na). The phosphonic acid-based chelating agent added to the polymer particles may be only a single phosphonic acid-based chelating agent or two or more phosphonic acid-based chelating agents.

The water content in the polymer particles when the phosphonic acid-based chelating agent is added thereto may be any water content in the range of 5% by mass or more and 100% by mass or less, but is preferably 15% by mass or more, more preferably 20% by mass or more, and still more preferably 25% by mass or more, while the water content is preferably 90% by mass or less, more preferably 80% by mass or less, and still more preferably 70% by mass or less, in view of more suitably achieving the effects of the present invention. Preferred ranges include from 5 to 90% by mass, from 5 to 80% by mass, from 5 to 70% by mass, from 15 to 90% by mass, from 15 to 80% by mass, from 15 to 70% by mass, from 20 to 90% by mass, from 20 to 80% by mass, from 20 to 70% by mass, from 25 to 90% by mass, from 25 to 80% by mass, and from 25 to 70% by mass. The water content in the polymer particles when the phosphonic acid-based chelating agent is added thereto can be adjusted by the below-described drying step (including dehydration), addition of water to the polymer particles, and the like.

Furthermore, the amount of the phosphonic acid-based chelating agent added may be any amount in the range of 0.055 parts by mass or more and 0.6 parts by mass or less per 100 parts by mass of the water-soluble ethylenically unsaturated monomer, but the amount is preferably 0.055 parts by mass or more, more preferably 0.056 parts by mass or more, and still more preferably 0.057 parts by mass or more, while the amount is preferably 0.6 parts by mass or less, more preferably 0.4 parts by mass or less, and still more preferably 0.3 parts by mass or less, in view of more suitably achieving the effects of the present invention. Preferred ranges include from 0.055 to 0.6 parts by mass, from 0.055 to 0.4 parts by mass, from 0.055 to 0.3 parts by mass, from 0.056 to 0.6 parts by mass, from 0.056 to 0.4 parts by mass, from 0.056 to 0.3 parts by mass, from 0.057 to 0.6 parts by mass, from 0.057 to 0.4 parts by mass, and from 0.057 to 0.3 parts by mass.

In the present invention, the phosphonic acid-based chelating agent may be added to the polymer particles before the step of surface-crosslinking after the polymerization step, or after the step of surface-crosslinking, or both before the step of surface-crosslinking after the polymerization step, and after the step of surface-crosslinking. In view of more suitably achieving the effects of the present invention, it is more preferred to add the phosphonic acid-based chelating agent to the polymer particles at least before the step of surface-crosslinking after the polymerization step. When the phosphonic acid-based chelating agent is added to the polymer particles both before and after the step of surface-crosslinking, it is only required that, during the addition at least one of before and after the step of surface-crosslinking, the water content in the polymer particles is in the range of 5% by mass or more and 100% by mass or less, and the amount of the phosphonic acid-based chelating agent added is in the range of 0.055 parts by mass or more and 0.6 parts by mass or less per 100 parts by mass of the water-soluble ethylenically unsaturated monomer; however, it is also preferred that, during the addition both before and after the step of surface-crosslinking, the water content in the polymer particles and the amount of the phosphonic acid-based chelating agent added fall in these ranges.

Examples of methods of adding the phosphonic acid-based chelating agent to the polymer particles include, but are not specifically limited to, a method in which the phosphonic acid-based chelating agent in a solid state, such as a powder, is mixed into the polymer particles; a method in which an aqueous solution of the phosphonic acid-based chelating agent is added dropwise to the polymer particles dispersed in an organic solvent; and a method in which an aqueous solution of the phosphonic acid-based chelating agent is sprayed to the surface of the polymer particles. Examples of methods of spraying an aqueous solution of the phosphonic acid-based chelating agent include using a spray nozzle device, and using an ultrasonic device with an ultrasonic vibrator, or a rotary atomizing centrifugal spray device. In the present invention, the method using a spray nozzle device is preferred. The spray nozzle device is not specifically limited as long as it is capable of spraying an aqueous solution, but is preferably a one fluid-type or two fluid-type spray having a spray pattern such as a flat spray, a hollow cone, or a full cone.

<Drying Step>

After the surface-crosslinking is performed as described above, the method may include a drying step of removing the water, the hydrocarbon dispersion medium, and the like by distillation, by adding external energy, such as heat. The polymer particles after the surface-crosslinking are dried to distill off the water and hydrocarbon dispersion medium to obtain water-absorbent resin particles. By controlling the treatment conditions for the drying step after the surface-crosslinking to adjust the amount of water to be removed (that is, adjust the water content in the polymer particles), the water content in the polymer particles when the phosphonic acid-based chelating agent is added thereto can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the step of surface-crosslinking with a surface-crosslinking agent is performed after the polymerization of the monomer by reversed phase suspension polymerization, the drying step by distillation is performed as described above, after the completion of the step of surface-crosslinking. Alternatively, the step of surface-crosslinking and the drying step may be performed simultaneously.

The water-absorbent resin particles of the present invention may contain additives suitable for its purpose. Examples of such additives include inorganic powders, surfactants, oxidizing agents, reducing agents, metal chelating agents (metal chelating agents different from the phosphonic acid-based chelating agent), radical chain inhibitors, antioxidants, and anti-bacterial agents. For example, when 0.05 to 5 parts by mass of amorphous silica as an inorganic powder is added to 100 parts by mass of the water-absorbent resin particles, the flowability of the water-absorbent resin particles can be further improved. The additives are preferably hydrophilic or water-soluble.

2. Water-Absorbent Resin Particles

By employing the method for producing water-absorbent resin particles of the present invention described above, it is possible to produce water-absorbent resin particles having gel stability against artificial urine containing iron, long-term gel stability against artificial urine, and yellowing resistance. Furthermore, by employing the method for producing water-absorbent resin particles of the present invention described above, it is also possible to achieve not only these properties, but also improved physiological saline retention capacity and improved physiological saline absorption capacity under a load of 4.14 kPa. More specifically, by employing the method for producing water-absorbent resin particles of the present invention, it is possible to suitably produce water-absorbent resin particles having, for example, the following properties (A) to (E):

(A) a physiological saline retention capacity of 20 g/g or more and 70 g/g or less;
(B) a physiological saline absorption capacity under a load of 4.14 kPa of 5 mL/g or more and 40 mL/g or less;
(C) a yellowness index of less than 25 after allowing the water-absorbent resin particles to stand for 7 days in an environment of 70°C and 90% relative humidity;
(D) a gel strength of 6000 N/m² or more after allowing a gel swollen 40-fold with artificial urine to stand for 14 hours in an environment of 37°C and 60% relative humidity; and

(E) a gel strength of 4000 N/m$^2$ or more after allowing a gel swollen 40-fold with artificial urine containing iron at an iron concentration of 10 ppm to stand for 2.5 hours in an environment of 25°C and 50% relative humidity.

The physiological saline retention capacity (A) of the water-absorbent resin particles of the present invention is preferably 20 g/g or more, and more preferably 30 g/g or more, while it is preferably 70 g/g or less, more preferably 50 g/g or less, still more preferably 45 g/g or less, and even more preferably 43 g/g or less. Preferred ranges include from 20 to 70 g/g, from 20 to 50 g/g, from 20 to 45 g/g, from 20 to 43 g/g, from 30 to 70 g/g, from 30 to 50 g/g, from 30 to 45 g/g, and from 30 to 43 g/g.

The physiological saline absorption capacity under a load of 4.14 kPa (B) of the water-absorbent resin particles of the present invention is preferably 10 mL/g or more, more preferably 15 mL/g or more, and still more preferably 18 mL/g or more, while it is preferably 30 mL/g or less. Preferred ranges include from 10 to 30 mL/g, from 15 to 30 mL/g, and from 18 to 30 mL/g.

The yellowness index (C) of the water-absorbent resin particles of the present invention after allowing to stand for 7 days in an environment of 70°C and 90% relative humidity is preferably less than 25, more preferably less than 20, and still more preferably less than 15. The lower limit of the yellowness index is, for example, zero.

The initial value of the yellowness index of the water-absorbent resin particles of the present invention is preferably less than 20, more preferably less than 15, and still more preferably less than 12. The lower limit of the yellowness index is, for example, zero.

The gel strength (D) of the water-absorbent resin particles of the present invention after allowing a gel swollen 40-fold with artificial urine to stand for 14 hours in an environment of 37°C and 60% relative humidity is preferably 6000 N/m$^2$ or more, more preferably 6500 N/m$^2$ or more, and still more preferably 7000 N/m$^2$ or more, while it is preferably 30000 N/m$^2$ or less, more preferably 20000 N/m$^2$ or less, still more preferably 15000 N/m$^2$ or less, and even more preferably 10000 N/m$^2$ or less. Preferred ranges include from 6000 to 30000 N/m$^2$, from 6000 to 20000 N/m$^2$, from 6000 to 15000 N/m$^2$, from 6000 to 10000 N/m$^2$, from 6500 to 30000 N/m$^2$, from 6500 to 20000 N/m$^2$, from 6500 to 15000 N/m$^2$, from 6500 to 10000 N/m$^2$, from 7000 to 30000 N/m$^2$, from 7000 to 20000 N/m$^2$, from 7000 to 15000 N/m$^2$, and from 7000 to 10000 N/m$^2$. The upper limit of the initial value of the gel strength is, for example, 20000 N/m$^2$.

The gel strength (E) of the water-absorbent resin particles of the present invention after allowing a gel swollen 40-fold with artificial urine containing iron at an iron concentration of 10 ppm to stand for 2.5 hours in an environment of 25°C and 50% relative humidity is preferably 4000 N/m$^2$ or more, and more preferably 4300 N/m$^2$ or more, while it is preferably 15000 N/m$^2$ or less, more preferably 10000 N/m$^2$ or less, and still more preferably 8000 N/m$^2$ or less. Preferred ranges include from 4000 to 15000 N/m$^2$.

Each of the properties (A) to (E) of the water-absorbent resin particles is measured by the method described in the Examples. The initial value of the yellowness index of the water-absorbent resin particles of the present invention is also measured by the method described in the Examples.

The water-absorbent resin particles of the present invention are formed of a crosslinked product of a polymer of a water-soluble ethylenically unsaturated monomer, that is, a crosslinked polymer having a structural unit derived from a water-soluble ethylenically unsaturated monomer.

The water-absorbent resin particles of the present invention are in a form in which fine particles (primary particles) are agglomerated (secondary particles). Examples of the shape of the primary particles include a substantially spherical shape, a crushed indefinite shape, and a flat shape. The water-absorbent resin particles of the present invention that are the secondary particles may have various shapes. Examples of the shape of the water-absorbent resin particles include a granular shape, a substantially spherical shape, a crushed indefinite shape, a flat shape, a fibrous shape, a flake shape, or a shape formed by agglomeration of such a resin. The water-absorbent resin particles preferably have, for example, a granular shape, a substantially spherical shape, a crushed indefinite shape, a fibrous shape, or a shape formed by agglomeration of such a resin.

The water-absorbent resin particles preferably have a median particle size of 200 μm or more, 250 μm or more, 280 μm or more, 300 μm or more, or 320 μm or more. From the same viewpoint, the median particle size is preferably 700 μm or less, 600 μm or less, 550 μm or less, 500 μm or less, 450 μm or less, or 400 μm or less. That is, the median particle size is preferably 200 to 700 μm, more preferably 200 to 600 μm, still more preferably 250 to 500 μm, even more preferably 300 to 450 μm, and still more preferably 320 to 400 μm.

The median particle size of the water-absorbent resin particles can be measured using JIS standard sieves; specifically, it is the value as measured by the method described in the Examples section.

The water-absorbent resin particles have a physiological saline absorption rate of preferably 20 seconds or more, more preferably 25 seconds or more, still more preferably 30 seconds or more. On the other hand, the physiological saline absorption rate is preferably 65 seconds or less, more preferably 60 seconds or less, and still more preferably 55 seconds or less. Preferred ranges include from 20 to 65 seconds and from 25 to 60 seconds.

The physiological saline absorption rate of the water-absorbent resin particles is the value as measured by the method described in the Examples section.

3. Absorbent Material and Absorbent Article

The water-absorbent resin particles of the present invention constitute an absorbent material used for hygienic materials, such as sanitary articles and disposable diapers, for example, and is suitably used for an absorbent article including the absorbent material.

The absorbent material of the present invention contains the water-absorbent resin particles of the present invention. The absorbent material may further contain hydrophilic fibers. Examples of the structure of the absorbent material include a sheet-like structure in which the water-absorbent resin particles are fixed on a nonwoven fabric or between a plurality of nonwoven fabrics; a mixed dispersion obtained by mixing the water-absorbent resin particles and hydrophilic fibers to give a uniform composition; a sandwich structure in which the water-absorbent resin particles are sandwiched between layered hydrophilic fibers; and a structure in which the water-absorbent resin particles and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The water-absorbent resin particles in the absorbent material of the present invention have a basis weight of 50 $g/m^2$ or more and 400 $g/m^2$ or less. The basis weight is preferably 100 $g/m^2$ or more, more preferably 120 $g/m^2$ or more, and still more preferably 140 $g/m^2$ or more, while the basis weight is preferably 300 $g/m^2$ or less, more preferably 250 $g/m^2$ or less, and still more preferably 200 $g/m^2$ or less.

The hydrophilic fibers may be, for example, at least one selected from the group consisting of finely ground wood pulp, cotton, cotton linter, rayon, cellulose acetate, polyamides, polyesters, and polyolefins. Examples include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamides, polyesters, and polyolefins. The hydrophilic fibers typically have an average fiber length of 0.1 to 10 mm. Alternatively, the average fiber length may be 0.5 to 5 mm.

The hydrophilic fibers in the absorbent material of present invention have a basis weight of 0 $g/m^2$ or more and 800 $g/m^2$ or less. The basis weight is preferably 100 $g/m^2$ or more, more preferably 120 $g/m^2$ or more, and still more preferably 140 $g/m^2$ or more, while the basis weight is preferably 700 $g/m^2$ or less, more preferably 600 $g/m^2$ or less, and still more preferably 500 $g/m^2$ or less.

The content of the water-absorbent resin particles in the absorbent material is preferably 5 to 100% by mass, more preferably 10 to 95% by mass, still more preferably 20 to 90% by mass, and even more preferably 30 to 80% by mass.

It is possible to obtain the absorbent article of the present invention by holding the absorbent material obtained using the water-absorbent resin particles of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride. The liquid-permeable sheet is preferably at least one selected from the group consisting of a thermally bonded nonwoven fabric, an air-through nonwoven fabric, a spunbond nonwoven fabric, and a spunbond/melt-blown/spunbond nonwoven fabric.

The liquid-permeable sheet has a basis weight of preferably 5 $g/m^2$ or more and 100 $g/m^2$ or less, more preferably 10 $g/m^2$ or more and 60 $g/m^2$ or less. The surface of the liquid-permeable sheet may be embossed or perforated in order to improve the liquid diffusibility. The embossing and perforating can be performed by known methods.

Examples of the liquid-impermeable sheet include a sheet made of a synthetic resin such as polyethylene, polypropylene, or polyvinyl chloride, a sheet made of a nonwoven fabric such as spunbond/melt-blown/spunbond (SMS) nonwoven fabric in which a water-resistant melt-blown nonwoven fabric is sandwiched between high-strength spunbond nonwoven fabrics, and a sheet made of a composite material of these synthetic resins and a nonwoven fabric (for example, a spunbond nonwoven fabric or a spunlace nonwoven fabric). As the liquid-impermeable sheet, a sheet made of a synthetic resin mainly containing a low-density polyethylene (LDPE) resin can also be used. The liquid-impermeable sheet may be, for example, a sheet made of a synthetic resin having a basis weight of 10 to 50 $g/m^2$.

The absorbent article preferably includes a laminate having an absorbent material containing the water-absorbent resin particles and core wraps sandwiching upper and lower portions of the absorbent material; a liquid-permeable sheet disposed on an upper surface of the laminate; and a liquid-impermeable sheet disposed on a surface of the laminate opposite to the liquid-permeable sheet side.

4. Additional Matters

The present specification includes at least the inventions as set forth in (1) to (9) below.

(1) A method for producing water-absorbent resin particles, comprising the steps of:

polymerizing a water-soluble ethylenically unsaturated monomer to obtain polymer particles; and
subjecting the polymer particles to surface-crosslinking,
wherein before and/or after the step of surface-crosslinking, a phosphonic acid-based chelating agent is added to the polymer particles having a water content of 5% by mass or more and 100% by mass or less, and
wherein an amount of the phosphonic acid-based chelating agent added is 0.055 parts by mass or more and 0.6 parts by mass or less per 100 parts by mass of the water-soluble ethylenically unsaturated monomer.

(2) The method according to (1) above, wherein the water content in the polymer particles is 20% by mass or more and 80% by mass or less.
(3) The method according to (1) or (2) above, wherein the amount of the phosphonic acid-based chelating agent added is 0.055 to 0.4 parts by mass, 0.055 to 0.3 parts by mass, 0.057 to 0.4 parts by mass, or 0.057 to 0.3 parts by mass, per 100 parts by mass of the water-soluble ethylenically unsaturated monomer.
(4) The method according to any one of (1) to (3) above, wherein the water content in the polymer particles when a surface-crosslinking agent is added thereto is 10 to 60% by mass, 10 to 40% by mass, or 10 to 35% by mass.
(5) Water-absorbent resin particles, the water-absorbent resin particles being a crosslinked product of a polymer of a water-soluble ethylenically unsaturated monomer,
wherein the water-absorbent resin particles have the following properties (A) to (E):

(A) a physiological saline retention capacity of 20 g/g or more and 70 g/g or less;
(B) a physiological saline absorption capacity under a load of 4.14 kPa of 5 mL/g or more and 40 mL/g or less;
(C) a yellowness index of less than 25 after allowing the water-absorbent resin particles to stand for 7 days in an environment of 70°C and 90% relative humidity;
(D) a gel strength of 6000 $N/m^2$ or more after allowing a gel swollen 40-fold with artificial urine to stand for 14 hours in an environment of 37°C and 60% relative humidity; and
(E) a gel strength of 4000 $N/m^2$ or more after allowing a gel swollen 40-fold with artificial urine containing iron at an iron concentration of 10 ppm to stand for 2.5 hours in an environment of 25°C and 50% relative humidity.

(6) The water-absorbent resin particles according to (5) above, wherein the physiological saline retention capacity is 20 to 50 g/g, 30 to 45 g/g, or 30 to 43 g/g.
(7) The water-absorbent resin particles according to (5) or (6) above, wherein the physiological saline absorption capacity under a load of 4.14 kPa is 10 to 30 mL/g, 15 to 30 mL/g, or 18 to 30 mL/g.
(8) The water-absorbent resin particles according to any one of (5) to (7) above, wherein the gel strength after allowing a gel swollen 40-fold with artificial urine to stand for 14 hours in an environment of 37°C and 60% relative humidity is 6000 to 20000 $N/m^2$, 6500 to 15000 $N/m^2$, or 7000 to 10000 $N/m^2$.
(9) The water-absorbent resin particles according to any one of (5) to (8) above, wherein the gel strength after allowing a gel swollen 40-fold with artificial urine containing iron at an iron concentration of 10 ppm to stand for 2.5 hours in an environment of 25°C and 50% relative humidity is 4000 to 15000 $N/m^2$.

Examples

The present invention will be hereinafter described in detail with reference to examples and comparative examples. However, the present invention is not limited to the examples.
The polymer particles (crosslinked polymer particles) obtained in the production example below and the water-absorbent resin particles obtained in the examples and comparative examples below were evaluated using the tests as described below. Unless otherwise indicated, the measurement was performed in an environment at a temperature of 25 ± 2°C and a relative humidity of 50 ± 10%.

<Example 1>

<Polymerization Step>

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.0736 g (0.272 mmol) of potassium persulfate as a water-soluble radical polymerization initiator and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry.

Separately, in another 500 mL inner volume beaker, 128.8 g (1.44 mol) of an 80.5% by mass aqueous solution of acrylic acid was placed as a water-soluble ethylenically unsaturated monomer, and 160.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0116 g (0.067 mmol) of ethylene glycol diglycidyl ether as an internal-crosslinking agent were added and dissolved to prepare a second-stage aqueous solution.

The separable flask system was cooled to 27°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give crosslinked polymer particles.

[Dehydration Step]

After the crosslinked polymer particles were obtained as described above, the flask was immersed in an oil bath set at 125°C to remove 183.8 g of water out of the system by azeotropic distillation of the water and n-heptane, while refluxing the n-heptane. Then, 4.37 g of a 3.0% by mass aqueous solution of ethylenediamine tetramethylene phosphonic acid•pentasodium salt was added with stirring. Here, the water content in the crosslinked polymer particles when the aqueous solution of ethylenediamine tetramethylene phosphonic acid•pentasodium salt was added thereto was 57% by mass. Then again, 67.6 g of water was removed out of the system by azeotropic distillation of the water and n-heptane, while refluxing the n-heptane.

<Step of Surface-Crosslinking>

Then, 4.42 g (0.507 mmol) of a 2% by mass aqueous solution of ethylene glycol diglycidyl ether was added to the flask as a surface-crosslinking agent, and the flask was maintained at 83°C for 2 hours. Here, the water content in the crosslinked polymer particles when the aqueous solution of ethylene glycol diglycidyl ether was added thereto was 28% by mass.

<Drying Step>

Then, the reaction mixture was dried by evaporating the n-heptane at 125°C, and the resulting product was subsequently passed through a sieve with a mesh size of 850 $\mu$m to give water-absorbent resin particles. 100 parts by mass of the water-absorbent resin particles were mixed with 0.5 parts by mass of amorphous silica (Tokusil NP-S from Oriental Silicas Corporation) to give 220.3 g of water-absorbent resin particles (1). The water-absorbent resin particles (1) had a physiological saline retention capacity of 41 g/g, a physiological saline absorption capacity under a load of 4.14 kPa of 20 mL/g, a physiological saline absorption rate of 35 seconds, and a median particle size of 338 $\mu$m.

<Example 2>

<Polymerization Step>

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.0184 g (0.068 mmol) of potassium persulfate and 0.092 g (0.339 mmol) of 2,2'-azobis(2-amidinopropane) dihydrochloride as water-soluble radical polymerization initiators, and 0.0046 g (0.026 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry.

Separately, in another 500 mL inner volume beaker, 128.8 g (1.44 mol) of an 80.5% by mass aqueous solution of acrylic acid was placed as a water-soluble ethylenically unsaturated monomer, and 160.0 g of a 27% by mass aqueous solution of sodium hydroxide was added dropwise, while cooling with ice water, to achieve 75 mol% neutralization. Then, 0.0258 g (0.095 mmol) of potassium persulfate and 0.129 g (0.475 mmol) of 2,2'-azobis(2-amidinopropane) dihydrochloride as water-soluble radical polymerization initiators, and 0.0116 g (0.067 mmol) of ethylene glycol diglycidyl ether as an internal-crosslinking agent were added and dissolved to prepare a second-stage aqueous solution.

The separable flask system was cooled to 27°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give crosslinked polymer particles.

[Dehydration Step]

After the crosslinked polymer particles were obtained as described above, the flask was immersed in an oil bath set at 125°C to remove 183.7 g of water out of the system by azeotropic distillation of the water and n-heptane, while refluxing the n-heptane. Then, 4.37 g of a 3.0% by mass aqueous solution of ethylenediamine tetramethylene phosphonic acid•pentasodium salt was added with stirring. Here, the water content in the crosslinked polymer particles when the aqueous solution of ethylenediamine tetramethylene phosphonic acid•pentasodium salt was added thereto was 57% by mass. Then again, 47.9 g of water was removed out of the system by azeotropic distillation of the water and n-heptane, while refluxing the n-heptane.

<Step of Surface-Crosslinking>

Then, 4.42 g (0.507 mmol) of a 2% by mass aqueous solution of ethylene glycol diglycidyl ether was added to the flask as a surface-crosslinking agent, and the flask was maintained at 83°C for 2 hours. Here, the water content in the crosslinked polymer particles when the aqueous solution of ethylene glycol diglycidyl ether was added thereto was 37% by mass.

<Drying Step>

Then, the reaction mixture was dried by evaporating the n-heptane at 125°C, and the resulting product was subsequently passed through a sieve with a mesh size of 850 $\mu$m to give water-absorbent resin particles. 100 parts by mass of the water-absorbent resin particles were mixed with 0.5 parts by mass of amorphous silica (Tokusil NP-S from Oriental Silicas Corporation) to give 219.7 g of water-absorbent resin particles (2). The water-absorbent resin particles (2) had a physiological saline retention capacity of 46 g/g, a physiological saline absorption capacity under a load of 4.14 kPa of 17 mL/g, a physiological saline absorption rate of 48 seconds, and a median particle size of 346 $\mu$m.

<Example 3>

The same procedure as in Example 1 was repeated except that in [Dehydration Step] of Example 1, the amount of water removed out of the system by azeotropic distillation of the n-heptane and water was changed from 67.6 g to 61.0 g, and the water content in the crosslinked polymer particles when the 2% by mass aqueous solution of ethylene glycol diglycidyl ether was added thereto as a surface-crosslinking agent was changed from 28% by mass to 31% by mass, to give 221.3 g of water-absorbent resin particles (3). Here, the water-absorbent resin particles (3) had a physiological saline retention capacity of 34 g/g, a physiological saline absorption capacity under a load of 4.14 kPa of 28 mL/g, a physiological saline absorption rate of 38 seconds, and a median particle size of 340 μm.

<Example 4>

The same procedure as in Example 1 was repeated except that in [Dehydration Step] of Example 1, 4.37 g of a 3.0% by mass aqueous solution of diethylenetriamine pentamethylene phosphonic acid•heptasodium salt was used instead of 4.37 g of the 3.0% by mass aqueous solution of ethylenediamine tetramethylene phosphonic acid•pentasodium salt, to give 220.2 g of water-absorbent resin particles (4). The water content in the crosslinked polymer particles when the 2% by mass aqueous solution of ethylene glycol diglycidyl ether was added thereto as a surface-crosslinking agent was 28% by mass. The water-absorbent resin particles (4) had a physiological saline retention capacity of 38 g/g, a physiological saline absorption capacity under a load of 4.14 kPa of 21 mL/g, a physiological saline absorption rate of 37 seconds, and a median particle size of 340 μm.

<Comparative Example 1>

The same procedure as in Example 1 was repeated except that in [Dehydration Step] of Example 1, the amount of water removed out of the system by azeotropic distillation of the n-heptane and water was changed from 183.8 g to 199.1 g, 4.86 g of a 4.5% by mass aqueous solution of diethylenetriaminepentaacetic acid•pentasodium salt (DTPA•5Na) was used instead of 4.37 g of the 3.0% by mass aqueous solution of ethylenediamine tetramethylene phosphonic acid•pentasodium salt, and the amount of water removed out of the system by azeotropic distillation of the n-heptane and water was changed from 67.6 g to 52.7 g, to give 222.0 g of water-absorbent resin particles (5). Here, the water content in the crosslinked polymer particles when the aqueous solution of diethylenetriaminepentaacetic acid•pentasodium salt was added thereto was 50% by mass, and the water content in the crosslinked polymer particles when the 2% by mass aqueous solution of ethylene glycol diglycidyl ether was added thereto as a surface-crosslinking agent was 28% by mass. The water-absorbent resin particles (5) had a physiological saline retention capacity of 41 g/g, a physiological saline absorption capacity under a load of 4.14 kPa of 19 mL/g, a physiological saline absorption rate of 35 seconds, and a median particle size of 363 μm.

<Production Example>

<Polymerization Step>

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.0736 g (0.272 mmol) of potassium persulfate as a water-soluble radical polymerization initiator and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry.

Separately, in another 500 mL inner volume beaker, 128.8 g (1.44 mol) of an 80.5% by mass aqueous solution of acrylic acid was placed as a water-soluble ethylenically unsaturated monomer, and 160.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0116 g (0.067 mmol) of ethylene glycol diglycidyl ether as an internal-crosslinking agent were added and dissolved to prepare a second-stage aqueous solution.

The separable flask system was cooled to 27°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give crosslinked polymer particles.

[Dehydration Step]

After the crosslinked polymer particles were obtained as described above, the flask was immersed in an oil bath set at 125°C to remove 247.17 g of water out of the system by azeotropic distillation of the water and n-heptane, while refluxing the n-heptane.

<Step of Surface-Crosslinking>

Then, 4.42 g (0.507 mmol) of a 2% by mass aqueous solution of ethylene glycol diglycidyl ether was added to the flask as a surface-crosslinking agent, and the flask was maintained at 83°C for 2 hours. Here, the water content in the crosslinked polymer particles when the aqueous solution of ethylene glycol diglycidyl ether was added thereto was 28% by mass.

<Drying Step>

Then, the reaction mixture was dried by evaporating the n-heptane at 125°C to remove 50.09 g of water out of the system. The resulting product was subsequently passed through a sieve with a mesh size of 850 μm to give 220.1 g of crosslinked polymer particles (A). Here, the water content in the crosslinked polymer particles (A) was 7% by mass.

<Comparative Example 2>

Triethylenetetraminehexaacetic acid (TTHA) as a chelating agent was added in the form of powder in an amount of 0.04 parts by mass to 20 parts by mass of the crosslinked polymer particles (A) obtained in the Production Example, and the mixture was mixed for 30 minutes using a cross-rotary mixer from Meiwa Kogyo Co., Ltd. (conditions: revolution speed 50 rpm, rotation speed 50 rpm), to give a mixture. Subsequently, 100 parts by mass of the mixture was mixed with 0.5 parts by mass of amorphous silica (Tokusil NP-S from Oriental Silicas Corporation) to give water-absorbent resin particles (6). The water-absorbent resin particles (6) had a physiological saline retention capacity of 40 g/g, a physiological saline absorption capacity under a load of 4.14 kPa of 21 mL/g, a physiological saline absorption rate of 36 seconds, and a median particle size of 344 μm.

<Example 5>

20 parts by mass of the crosslinked polymer particles (A) obtained in the Production Example were weighed into an 11 cm inner diameter, cylindrical round-bottomed separable flask equipped with an anchor-type stirring blade made of a fluororesin. Next, while stirring at 300 rpm, 0.40 parts by mass of a 3.0% by mass aqueous solution of ethylenediamine tetramethylene phosphonic acid•pentasodium salt was collected with a 3 mL syringe, whose tip was then attached to Fine Atomizer Oral (Yoshikawa Kasei Co., Ltd.), and sprayed into the separable flask for 1 second, stirred for 10 minutes, and then heated at 100°C for 30 minutes to give a mixture. 100 parts by mass of the mixture was mixed with 0.5 parts by mass of amorphous silica (Tokusil NP-S from Oriental Silicas Corporation) to give water-absorbent resin particles (7). The water-absorbent resin particles (7) had a physiological saline retention capacity of 40 g/g, a physiological saline absorption capacity under a load of 4.14 kPa of 21 mL/g, a physiological saline absorption rate of 36 seconds, and a median particle size of 344 μm.

<Example 6>

The same procedure as in Example 1 was repeated except that in [Dehydration Step] of Example 1, the amount of water removed out of the system by azeotropic distillation of the n-heptane and water was changed from 183.8 g to 242.8 g, and the water content in the crosslinked polymer particles when the aqueous solution of ethylenediamine tetramethylene phosphonic acid•pentasodium salt was added thereto was changed from 57% by mass to 30% by mass; and the amount of water removed out of the system by azeotropic distillation of the n-heptane and water was changed from 67.6 g to 8.6 g, to give 221.5 g of water-absorbent resin particles (8). The water content in the crosslinked polymer particles when the 2% by mass aqueous solution of ethylene glycol diglycidyl ether was added thereto as a surface-crosslinking agent was 28% by mass. The water-absorbent resin particles (8) had a physiological saline retention capacity of 38 g/g, a physiological saline absorption capacity under a load of 4.14 kPa of 21 mL/g, a physiological saline absorption rate of 35 seconds, and a median particle size of 358 μm.

<Example 7>

The same procedure as in Example 1 was repeated except that in [Dehydration Step] of Example 1, the amount of water removed out of the system by azeotropic distillation of the n-heptane and water was changed from 183.8 g to 133.6 g, and the water content in the crosslinked polymer particles when the aqueous solution of ethylenediamine tetramethylene phosphonic acid•pentasodium salt was added thereto was changed from 57% by mass to 80% by mass; and the amount of water removed out of the system by azeotropic distillation of the n-heptane and water was changed from 67.6 g to 117.8 g, to give 224.5 g of water-absorbent resin particles (9). The water content in the crosslinked polymer particles when the 2% by mass aqueous solution of ethylene glycol diglycidyl ether was added thereto as a surface-crosslinking agent was 28% by mass. The water-absorbent resin particles (9) had a physiological saline retention capacity of 38 g/g, a physiological saline absorption capacity under a load of 4.14 kPa of 21 mL/g, a physiological saline absorption rate of 35 seconds, and a median particle size of 356 $\mu$m.

<Comparative Example 3>

The same procedure as in Example 1 was repeated except that in [Dehydration Step] of Example 1, 4.37 g of a 3.0% by mass aqueous solution of ethylenediamine tetramethylene phosphonic acid•pentasodium salt was added to the crosslinked polymer particles obtained without removing water out of the system by azeotropic distillation, and the water content in the crosslinked polymer particles when the aqueous solution of ethylenediamine tetramethylene phosphonic acid•pentasodium salt was added thereto was changed from 57% by mass to 141% by mass; and the amount of water removed out of the system by azeotropic distillation of the n-heptane and water was changed from 67.6 g to 251.4 g, to give 222.0 g of water-absorbent resin particles (10). The water content in the crosslinked polymer particles when the 2% by mass aqueous solution of ethylene glycol diglycidyl ether was added thereto as a surface-crosslinking agent was 28% by mass. The water-absorbent resin particles (10) had a physiological saline retention capacity of 40 g/g, a physiological saline absorption capacity under a load of 4.14 kPa of 19 mL/g, a physiological saline absorption rate of 36 seconds, and a median particle size of 400 $\mu$m.

<Example 8>

The same procedure as in Example 2 was repeated except that in [Dehydration Step] of Example 2, 4.37 g of a 5.0% by mass aqueous solution of ethylenediamine tetramethylene phosphonic acid•pentasodium salt was used instead of 4.37 g of the 3.0% by mass aqueous solution of ethylenediamine tetramethylene phosphonic acid•pentasodium salt, and the amount of water removed out of the system by azeotropic distillation of the n-heptane and water was changed from 47.9 g to 47.8 g, to give 219.5 g of water-absorbent resin particles (11). The water content in the crosslinked polymer particles when the 2% by mass aqueous solution of ethylene glycol diglycidyl ether was added thereto as a surface-crosslinking agent was 37% by mass. The water-absorbent resin particles (11) had a physiological saline retention capacity of 45 g/g, a physiological saline absorption capacity under a load of 4.14 kPa of 20 mL/g, a physiological saline absorption rate of 45 seconds, and a median particle size of 346 $\mu$m.

<Example 9>

The same procedure as in Example 5 was repeated except that in Example 5, 0.28 parts by mass of a 32.0% by mass aqueous solution of ethylenediamine tetramethylene phosphonic acid•pentasodium salt was used instead of 0.40 parts by mass of the 3.0% by mass aqueous solution of ethylenediamine tetramethylene phosphonic acid•pentasodium salt, to give water-absorbent resin particles (12). The water-absorbent resin particles (12) had a physiological saline retention capacity of 40 g/g, a physiological saline absorption capacity under a load of 4.14 kPa of 18 mL/g, a physiological saline absorption rate of 38 seconds, and a median particle size of 344 $\mu$m.

<Comparative Example 4>

The same procedure as in Example 1 was repeated except that in [Dehydration Step] of Example 1, 1.31 g of a 0.5% by mass aqueous solution of ethylenediamine tetramethylene phosphonic acid•pentasodium salt was used instead of 4.37 g of the 3.0% by mass aqueous solution of ethylenediamine tetramethylene phosphonic acid•pentasodium salt, and the amount of water removed out of the system by azeotropic distillation of the n-heptane and water was changed from 67.6 g to 64.7 g, to give 219.0 g of water-absorbent resin particles (13). The water content in the crosslinked polymer particles when the 2% by mass aqueous solution of ethylene glycol diglycidyl ether was added thereto as a surface-crosslinking agent was 28% by mass. The water-absorbent resin particles (13) had a physiological saline retention capacity of 42 g/g, a physiological saline absorption capacity under a load of 4.14 kPa of 18 mL/g, a physiological saline absorption rate of 37 seconds, and a median particle size of 366 μm.

<Water Content in Polymer Particles>

The water content in the polymer particles was calculated as follows. Using an amount of water W1 (g) contained in the aqueous solution used to produce the polymer particles in [Polymerization Step], an amount of water W2 (g) contained in the aqueous solution of the additive added into the system in [Dehydration Step], an amount of water W3 (g) contained in the aqueous solution of the surface-crosslinking agent added into the system in [Step of Surface-Crosslinking], an amount of water W4 (g) removed out of the system by azeotropic distillation of the n-heptane and water in [Dehydration Step], an amount of water W5 (g) removed out of the system by azeotropic distillation of the n-heptane and water in [Step of Surface-Crosslinking] and [Drying Step], and an amount M1 (g) of the water-soluble ethylenically unsaturated monomer contained in the aqueous solution used to produce the polymer particles in [Polymerization Step], the water content in the polymer particles was calculated based on the following equation:

Water content (% by mass) in polymer particles = (W1 + W2 + W3 - W4 - W5) × 100/M1

<Median Particle Size (Particle Size Distribution)>

10 g of the water-absorbent resin particles were screened using a sonic vibration type screening measuring device (Robot Sifter RPS-01, manufactured by Seishin Enterprise Co., Ltd), JIS standard sieves with mesh sizes of 850 μm, 600 μm, 500 μm, 425 μm, 300 μm, 250 μm and 180 μm, and a receiving tray. The mass of the particles remaining on each sieve was calculated as the mass percentage relative to the total mass. The mass percentage of the particles remaining on each sieve was integrated in descending order of particle size. In this manner, the relationship between the sieve mesh size and the cumulative value of the mass percentage of the particles remaining on each sieve was plotted on logarithmic probability paper. The plots on the probability paper were connected with straight lines, and a particle size equivalent to a 50% by mass cumulative mass percentage was determined as the median particle size.

<Physiological Saline Absorption Rate>

50 ± 0.1 g of physiological saline placed in a 100 ml beaker was adjusted to a temperature of 25 ± 0.2°C in a thermostat and then stirred with a magnetic stirrer bar (8 mm ϕ × 30 mm, no ring) at a rotation speed of 600 rpm to form a vortex. 2.0 ± 0.002 g of the water-absorbent resin particles were added at once into the physiological saline, and the time (seconds) from the addition of the water-absorbent resin particles to the point at which the vortex disappeared and the liquid surface became flat was measured. The measured time was determined as the physiological saline absorption rate of the water-absorbent resin particles.

<Physiological Saline Retention Capacity>

2.0 g of the water-absorbent resin particles were weighed into a cotton bag (Cottonbroad No. 60, 100 mm in width × 200 mm in length), and the cotton bag was placed in a 500 mL beaker. 500 g of a 0.9% by mass aqueous solution of sodium chloride (physiological saline) was poured at once into the cotton bag containing the water-absorbent resin particles so as to prevent formation of unswollen lumps, then the top of the cotton bag was closed with a rubber band, and the cotton bag was allowed to stand for 30 minutes to cause the water-absorbent resin particles to swell. After 30 minutes, the cotton bag was dehydrated for 1 minute using a dehydrator (product number: H-122 from Kokusan Co., Ltd.) set at a centrifugal force of 167 G, and a mass Wd (g) of the cotton bag containing the swollen gel after dehydration was measured. The same procedure was repeated without adding the water-absorbent resin particles, and an empty mass We (g) of the cotton bag upon wetting was measured. The physiological saline retention capacity was calculated according to the following equation:

$$\text{Physiological saline retention capacity (g/g)} = [\text{Wd} - \text{We}]/2.0$$

<Physiological Saline Absorption Capacity Under a Load of 4.14 kPa>

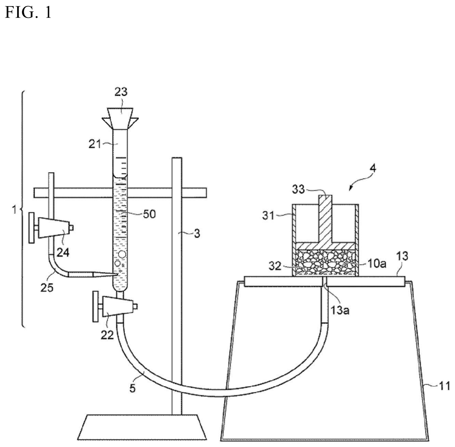

The physiological saline absorption capacity under a load of 4.14 kPa was measured using the measurement apparatus as schematically shown in Fig. 1. Two measurements were made for one type of water-absorbent resin particles, and the average value was obtained. The measurement apparatus includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through-hole 13a with a diameter of 2 mm formed in the central portion thereof, and is supported by the variable-height stand 11. The through-hole 13a of the measurement table 13 and the cock 22 of the burette unit 1 are connected through the conduit 5. The conduit 5 has an inner diameter of 6 mm.

The measurement unit 4 includes a cylinder 31 made of plexiglas, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 μm (200 mesh). The weight 33 has a diameter of 19 mm and a mass of 119.6 g and, as described later, can apply a load of 4.14 kPa (0.6 psi) to water-absorbent resin particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Subsequently, the upper opening of the burette tube 21 was sealed with the rubber stopper 23, and then the cock 22 and the cock 24 were opened. The conduit 5 was filled with the 0.9% by mass saline 50 to prevent air bubbles from entering. The height of the measurement table 13 was adjusted so that the height of the surface of the 0.9% by mass saline reached into the through-hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at 0 seconds).

In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was placed such that the central portion thereof aligned with the conduit opening at the central portion of the measurement table 13. An amount of reduction Wc (mL) in the physiological saline in the burette tube 21 at 60 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 4.14 kPa of the water-absorbent resin particles 10a was calculated using the following equation:

Physiological saline absorption capacity (mL/g) under a load of 4.14 kPa = Wc (mL)/mass (g) of the water-absorbent resin particles

<Evaluation of Stability of Gel after Absorption of Artificial Urine Containing Iron>

Artificial urine containing iron (iron concentration: 10 ppm) of the following composition was absorbed into the water-absorbent resin particles, and the stability of the gel obtained after the absorption by the water-absorbent resin particles (swollen gel) was evaluated.

(Preparation of Artificial Urine Containing Iron)

Artificial urine containing iron of the following composition was prepared.

Urea: 20.0 g
Sodium chloride: 8.0 g
Calcium chloride dihydrate: 0.3 g
Magnesium sulfate heptahydrate: 0.8 g
L(+)- ascorbic acid: 0.2 g
Iron(II) sulfate heptahydrate: 0.05 g
Ion-exchanged water: 970.9 g

(Preparation of Swollen Gel)

39.0 g of the artificial urine containing iron was weighed into a 100 mL inner volume beaker. A magnetic stirrer bar (8 mm φ × 30 mm, no ring) was introduced into the beaker and placed on a magnetic stirrer (HS-30D from Iuchi Co., Ltd.), and then the magnetic stirrer bar was rotated at 600 rpm. Subsequently, 1.00 g of the water-absorbent resin particles were added into the beaker under stirring, and stirred until the rotating vortex disappeared and the liquid surface became horizontal. In this manner, a swollen gel for use as a measurement sample was prepared. Immediately after the preparation of the swollen gel, the beaker containing the swollen gel was covered with a wrap (Diawrap from Mitsubishi Chemical Corporation). The beaker containing the swollen gel covered with the wrap was allowed to stand for 2.5 hours in a room at a temperature of 25 ± 2°C and a relative humidity of 50 ± 10%, then the wrap was removed from the beaker containing the swollen gel, and the gel strength was measured by the below-described method.

(Measurement of Gel Strength)

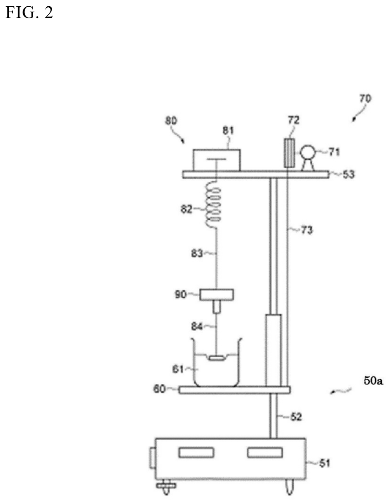

The gel strength after each standing time at each temperature was measured using an apparatus having the measurement principle as shown in Fig. 2. The apparatus shown in Fig. 2 includes a support unit 50a, a movable mount plate 60, a driving unit 70 for driving the movable mount plate 60, and a measurement unit 80. In the support unit 50a, a stand 53 is fixed to an upper portion of a column 52 standing on a column mount 51. The movable mount plate 60 is attached to the column 52 to be vertically movable. A measurement sample (gel) 61 can be mounted on the movable mount plate 60. A pulse motor 71 is mounted on the stand 53, and rotating a pulley 72 causes the movable mount plate 60 to vertically move via a wire 73. In the measuring unit 80, a disk-equipped pressure-sensitive shaft 84 is attached to a load cell 81 for measuring a strain caused by deformation, via a precision spring 82 and a connecting shaft 83. The disk-equipped pressure-sensitive shaft 84 has a disk at its tip. Depending on the measurement conditions, the diameter of the disk can vary. A weight 90 can be mounted on an upper portion of the disk-equipped pressure-sensitive shaft 84. The operation principle of the apparatus for measuring gel strength is as follows. An upper portion of the precision spring 82 is fixed to the load cell 81 (stress detector), and a lower portion of the precision spring 82 is connected to the disk-equipped pressure-sensitive shaft 84 with the predetermined weight 90 mounted thereon and suspended vertically. The movable mount plate 60 on which the measurement sample 61 is placed is raised at a constant speed by the rotation of the pulse motor 71. A load is applied at a constant speed to the measurement sample 61 via the precision spring 82, the strain caused by deformation is measured by the load cell 81, and the hardness is measured and calculated. The gel strength value ($N/m^2$) was measured using Curdmeter-MAX (product number: ME-500 from Asuka Kiki Co., Ltd.), using a 16 mm $\phi$ disk for the disk-equipped pressure-sensitive shaft 84, a load of 400 g, a speed of 7 seconds/inch, and the viscous mode setting.

<Evaluation of Long-Term Stability of Gel after Absorption of Artificial Urine>

Artificial urine of the following composition was absorbed into the water-absorbent resin particles, and the long-term stability of the gel obtained after the absorption by the water-absorbent resin particles (swollen gel) was evaluated.

(Preparation of Artificial Urine)

Artificial urine of the following composition was prepared.

Urea: 20.0 g
Sodium chloride: 8.0 g
Calcium chloride dihydrate: 0.3 g
Magnesium sulfate heptahydrate: 0.8 g
L(+)- ascorbic acid: 0.2 g
Ion-exchanged water: 970.9 g

(Preparation of Swollen Gel)

The same procedure as in <Evaluation of Stability of Gel after Absorption of Artificial Urine Containing Iron> was repeated except that in <Test on Gel after Absorption of Artificial Urine Containing Iron>, the above-described artificial urine was used instead of the artificial urine containing iron, and the beaker containing the swollen gel was allowed to stand for 14 hours in a constant temperature and humidity machine (LHU-113 from Espec Corporation) set at a temperature of 37 ± 2°C and a relative humidity of 60 ± 10%, instead of being allowed to stand for 2.5 hours in a room at a temperature of 25 ± 2°C and a relative humidity of 50 ± 10%, to prepare a swollen gel.

(Measurement of Gel Strength)

The temperature of the swollen gel was adjusted to 25 ± 2°C, and the gel strength was measured as in <Evaluation of Stability of Gel after Absorption of Artificial Urine Containing Iron>.

<Yellowing Test under High Temperature and High Humidity (Measurement of Yellowness Index)>

2.0 g of the water-absorbent resin particles were placed in a glass measurement container with an inner diameter of 3 cm. The yellowness index of the water-absorbent resin particles was measured with a colorimeter (Color Meter ZE6000 from Nippon Denshoku Industries Co., Ltd.) having the tristimulus values X, Y, and Z of a colorimetric color difference meter calibrated with a standard white plate. From the obtained X, Y, and Z (tristimulus values) of the water-absorbent resin particles, the yellowness index was calculated based on the following equation and used as the initial value.

$$\text{Yellowness index} = 100\,(1.28X - 1.06Z)/Y$$

Coloration of the water-absorbent resin particles with time was tested as follows. 2.0 g of the water-absorbent resin particles were placed uniformly in a glass petri dish with an inner diameter of 3 cm and a depth of 1 cm, and the container was stored for 7 days in a constant temperature and humidity machine (LHU-113 from Espec Corporation) set at a temperature of 70 $\pm$ 2°C and a relative humidity of 90 $\pm$ 2%. Then, the container was removed from the constant temperature and humidity chamber and allowed to stand for a while to cool to room temperature. The entire amount of the water-absorbent resin particles in the container was placed in a glass measurement container with an inner diameter of 3 cm, and the yellowness index of the water-absorbent resin particles was measured with a colorimeter (Color Meter ZE6000 from Nippon Denshoku Industries Co., Ltd.). From the obtained X, Y, and Z (tristimulus values) of the water-absorbent resin particles, the yellowness index was calculated based on the equation shown above.

[Table 1]

| Sample | | Chelating agent | | | Performance of water-absorbent resin particles | | | | Yellowing resistance | | Long-term gel stability against artificial urine | Gel stability against artificial urine containing iron |
|---|---|---|---|---|---|---|---|---|---|---|---|---|
| | | Type | After polymerization | | Physiological saline retention capacity [g/g] | Physiological saline absorption capacity under a load of 4.14 kPa [mL/g] | Physiological saline absorption rate [sec] | Median particle size [μm] | Yellowness index [-] | | after 14 hours | after 2.5 hours |
| | | | Amount added [ppm] | Water content [%] during addition | | | | | Initial value | Day 7 | Gel strength [N/m²] | Gel strength [N/m²] |
| Ex.1 | Water-absorbent resin particles(1) | EDTMP・5Na | 600 | 57 | 41 | 20 | 35 | 338 | 10 | 13 | 8641 | 5681 |
| Ex.2 | Water-absorbent resin particles(2) | EDTMP・5Na | 600 | 57 | 46 | 17 | 48 | 346 | 11 | 13 | 7154 | 4973 |
| Ex.3 | Water-absorbent resin particles(3) | EDTMP・5Na | 600 | 57 | 34 | 28 | 38 | 340 | 10 | 12 | 8782 | 6688 |
| Ex.4 | Water-absorbent resin particles(4) | DTPMP・7Na | 600 | 57 | 38 | 21 | 37 | 340 | 10 | 14 | 7604 | 6706 |
| Comp. Ex.1 | Water-absorbent resin particles(5) | DTPA・5Na | 1000 | 50 | 41 | 19 | 35 | 363 | 6 | 9 | 8615 | 1282 |
| Comp. Ex.2 | Water-absorbent resin particles(6) | TTHA | 2000 | 7 | 40 | 21 | 36 | 344 | 10 | 11 | 8954 | 1192 |
| Ex.5 | Water-absorbent resin particles(7) | EDTMP・5Na | 600 | 7 | 40 | 21 | 36 | 344 | 12 | 18 | 8212 | 6014 |
| Ex.6 | Water-absorbent resin particles(8) | EDTMP・5Na | 600 | 30 | 38 | 21 | 35 | 358 | 10 | 11 | 7628 | 6774 |
| Ex.7 | Water-absorbent resin particles(9) | EDTMP・5Na | 600 | 80 | 38 | 21 | 35 | 356 | 10 | 11 | 6820 | 5457 |
| Comp. Ex.3 | Water-absorbent resin particles(10) | EDTMP・5Na | 600 | 141 | 40 | 19 | 36 | 400 | 8 | 13 | 6694 | 0 |
| Ex.8 | Water-absorbent resin particles(11) | EDTMP・5Na | 1000 | 57 | 45 | 20 | 45 | 346 | 3 | 13 | 7340 | 4461 |
| Ex.9 | Water-absorbent resin particles(12) | EDTMP・5Na | 5000 | 7 | 40 | 18 | 38 | 344 | 10 | 18 | 6581 | 4206 |
| Comp. Ex.4 | Water-absorbent resin particles(13) | EDTMP・5Na | 30 | 57 | 42 | 18 | 37 | 366 | 10 | 29 | 8567 | 5876 |

Reference Signs List

1: burette unit
3: clamp
4: measurement unit
5: conduit
10a: water-absorbent resin particles
11: stand
13: measurement table
13a: through-hole
15: nylon mesh sheet
21: burette tube
22: cock
23: rubber stopper
24: cock
25: air inlet tube
31: cylinder
32: polyamide mesh
33: weight
50: saline
50a: support unit
51: column mount
52: column
53: stand
60: movable mount plate
61: measurement sample
70: drive unit
71: pulse motor
72: pulley

73: wire
80: measurement unit
81: load cell
82: precision spring
83: connecting shaft
84: disk-equipped pressure-sensitive shaft
90: weight

**Claims**

1. A method for producing water-absorbent resin particles, comprising the steps of:

   polymerizing a water-soluble ethylenically unsaturated monomer to obtain polymer particles; and
   subjecting the polymer particles to surface-crosslinking,
   wherein before and/or after the step of surface-crosslinking, a phosphonic acid-based chelating agent is added to the polymer particles having a water content of 5% by mass or more and 100% by mass or less, and
   wherein an amount of the phosphonic acid-based chelating agent added is 0.055 parts by mass or more and 0.6 parts by mass or less per 100 parts by mass of the water-soluble ethylenically unsaturated monomer.

2. The method according to claim 1, wherein the water content in the polymer particles is 20% by mass or more and 80% by mass or less.

3. The method according to claim 1 or 2, wherein the water-absorbent resin particles have the following properties (A) to (E):

   (A) a physiological saline retention capacity of 20 g/g or more and 70 g/g or less;
   (B) a physiological saline absorption capacity under a load of 4.14 kPa of 5 mL/g or more and 40 mL/g or less;
   (C) a yellowness index of less than 25 after allowing the water-absorbent resin particles to stand for 7 days in an environment of 70°C and 90% relative humidity;
   (D) a gel strength of 6000 N/m$^2$ or more after allowing a gel swollen 40-fold with artificial urine to stand for 14 hours in an environment of 37°C and 60% relative humidity; and
   (E) a gel strength of 4000 N/m$^2$ or more after allowing a gel swollen 40-fold with artificial urine containing iron at an iron concentration of 10 ppm to stand for 2.5 hours in an environment of 25°C and 50% relative humidity.

4. Water-absorbent resin particles, the water-absorbent resin particles being a crosslinked product of a polymer of a water-soluble ethylenically unsaturated monomer,
   wherein the water-absorbent resin particles have the following properties (A) to (E):

   (A) a physiological saline retention capacity of 20 g/g or more and 70 g/g or less;
   (B) a physiological saline absorption capacity under a load of 4.14 kPa of 5 mL/g or more and 40 mL/g or less;
   (C) a yellowness index of less than 25 after allowing the water-absorbent resin particles to stand for 7 days in an environment of 70°C and 90% relative humidity;
   (D) a gel strength of 6000 N/m$^2$ or more after allowing a gel swollen 40-fold with artificial urine to stand for 14 hours in an environment of 37°C and 60% relative humidity; and
   (E) a gel strength of 4000 N/m$^2$ or more after allowing a gel swollen 40-fold with artificial urine containing iron at an iron concentration of 10 ppm to stand for 2.5 hours in an environment of 25°C and 50% relative humidity.

5. An absorbent material comprising the water-absorbent resin particles according to claim 4.

6. An absorbent article comprising the absorbent material according to claim 5.

FIG. 1

FIG. 2

## INTERNATIONAL SEARCH REPORT

| International application No. |
| --- |
| **PCT/JP2024/022475** |

**A. CLASSIFICATION OF SUBJECT MATTER**

*C08J 3/12*(2006.01)i; *A61F 13/15*(2006.01)i; *A61F 13/53*(2006.01)i
FI: C08J3/12 A; A61F13/15 320; A61F13/53 300

According to International Patent Classification (IPC) or to both national classification and IPC

**B. FIELDS SEARCHED**

Minimum documentation searched (classification system followed by classification symbols)

A61F13/15; A61F13/53; B01J20/00-20/28; C08J3/00-3/28; C08L1/00-101/14; C08K3/00-13/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 1-275661 A (KAO CORPORATION) 06 November 1989 (1989-11-06) entire text | 1-6 |
| A | WO 2014/088012 A1 (NIPPON SHOKUBAI CO., LTD.) 12 June 2014 (2014-06-12) entire text | 1-6 |
| A | WO 2015/053372 A1 (NIPPON SHOKUBAI CO., LTD.) 16 April 2015 (2015-04-16) entire text | 1-6 |
| A | WO 2018/062539 A1 (NIPPON SHOKUBAI CO., LTD.) 05 April 2018 (2018-04-05) entire text | 1-6 |
| A | JP 8-52203 A (KAO CORPORATION) 27 February 1996 (1996-02-27) entire text | 1-6 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **03 September 2024** | **17 September 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

| International application No. |
| --- |
| **PCT/JP2024/022475** |

| Patent document cited in search report | Publication date (day/month/year) | Patent family member(s) | Publication date (day/month/year) |
| --- | --- | --- | --- |
| JP 1-275661 A | 06 November 1989 | (Family: none) | |
| WO 2014/088012 A1 | 12 June 2014 | US 2015/0307667 A1 entire text EP 2927266 A1 CN 104822740 A KR 10-2015-0091363 A | |
| WO 2015/053372 A1 | 16 April 2015 | EP 3056268 A1 entire text KR 10-2016-0068768 A CN 105658323 A | |
| WO 2018/062539 A1 | 05 April 2018 | US 2019/0338082 A1 entire text EP 3521376 A1 CN 110023412 A KR 10-2019-0077359 A | |
| JP 8-52203 A | 27 February 1996 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H3227301 A